# EUROPEAN PATENT SPECIFICATION

(11) **EP 3 761 988 B1**
(45) Date of publication and mention of the grant of the patent: **09.12.2026**
(21) Application number: 19763753.1
(22) Date of filing: 06.03.2019
(51) Int. Cl.: A61K 31/5377, A61K 31/4436, A61K 31/593, A61K 9/00, A61P 17/00, A61P 17/06, A61N 5/06, A61K 45/06

(54) **COMPOSITIONS AND METHODS FOR TREATING HYPERPROLIFERATIVE SKIN DISORDERS**
ZUSAMMENSETZUNGEN UND VERFAHREN ZUR BEHANDLUNG HYPERPROLIFERATIVER HAUTKRANKHEITEN
COMPOSITIONS ET MÉTHODES DE TRAITEMENT DES TROUBLES CUTANÉS HYPERPROLIFÉRATIFS

(30) Priority: 07.03.2018 US 201862639742 P
(43) Date of publication of application: 13.01.2021
(73) Proprietor: ATNX SPV, LLC, Toronto, Ontario M5J 2S1 (CA)
(72) Inventor: LAU, Johnson Yiu-Nam, Houston, Texas 77057 (US); VERONE-BOYLE, Alissa Rae, Kenmore, New Jersey 14217 (US); WONG, Chun-Ho, Fo Tan, Shatin, Hong Kong (CN); BU, Yahao, Williamsville, New York 14221 (US); CUTLER, Murray John, Fort Erie, Ontario L2A 3Y2 (CA); BELKO, Krista Elizabeth, Clarence Center, New York 14032 (US); KWAN, Min-Fun Rudolf, Summit, New Jersey 07901 (US)
(74) Representative: J A Kemp LLP
(86) International application number: PCT/US2019/021037
(87) International publication number: WO 2019/173533

(56) References cited:
- WO-A1-2014/194133
- WO-A2-2016/193441
- JP-A- H01 252 229
- US-A1- 2012 270 874
- US-A1- 2017 196 949
- US-B2- 7 939 529
- GAUKROGER J ET AL: "Cytotoxicity of etietinate and vindesine", JOURNAL OF ETHNOPHARMACOLOGY, ELSEVIER IRELAND LTD, IE, vol. 17, no. 3, 1 September 1986 (1986-09-01), pages 298, XP023826910, ISSN: 0378-8741, [retrieved on 19860901], DOI: 10.1016/0378-8741(86)90123-6
- HERMANN T W ET AL: "The retinoid X receptor agonist bexarotene (Targretin) synergistically enhances the growth inhibitory activity of cytotoxic drugs in non-small cell lung cancer cells", LUNG CANCER, ELSEVIER, AMSTERDAM, NL, vol. 50, no. 1, 1 October 2005 (2005-10-01), pages 9 - 18, XP027774336, ISSN: 0169-5002, [retrieved on 20051001]
- TORSEKAR R ET AL: "Topical therapies in psoriasis", vol. 8, no. 4, 1 January 2017 (2017-01-01), pages 235 - 245, XP055851764, ISSN: 2229-5178, Retrieved from the Internet <URL:https://www.ncbi.nlm.nih.gov/pmc/articles/PMC5518573/pdf/IDOJ-8-235.pdf> DOI: 10.4103/2229-5178.209622
- PETER C N VAN DE KERKHOF: "An Update on Topical Therapies for Mild-Moderate Psoriasis", vol. 33, no. 1, 1 January 2015 (2015-01-01), pages 73 - 77, XP009522720, ISSN: 0733-8635, Retrieved from the Internet <URL:https://api.elsevier.com/content/article/PII:S0733863514001120?httpAccept=text/plain> [retrieved on 20141128], DOI: 10.1016/J.DET.2014.09.006
- HALVERSTAM C P ET AL: "Nonstandard and off-label therapies for psoriasis", CLINICS IN DERMATOLOGY, J.B. LIPPINCOTT, PHILADELPHIA, PA, US, vol. 26, no. 5, 1 September 2008 (2008-09-01), pages 546 - 553, XP024100245, ISSN: 0738-081X, [retrieved on 20080826], DOI: 10.1016/J.CLINDERMATOL.2007.10.023
- KINEX PHARMACEUTICALS: "Kinex Pharmceuticals announces allowance of the Investigational New Drug (IND) application for KX2-391 ointment by the United States Food and Drug Administration (FDA)", 24 July 2014 (2014-07-24), pages 1 - 3, XP055642704, Retrieved from the Internet <URL:https://www.prnewswire.com/news-releases/kinex-pharmaceuticals-announces-allowance-of-the-investigational-new-drug-ind-application-for-kx2-391-ointment-by-the-united-states-food-and-drug-administration-fda-268466522.html>
- LESLEY J SCOTT , CHRISTOPHER J DUNN , KAREN L GOA : "Calcipotriol ointment", AMERICAN JOURNAL OF CLINICAL DERMATOLOGY, vol. 2, no. 2, 22 August 2012 (2012-08-22), pages 95 - 120, XP009522812, DOI: 10.2165/00128071-200102020-00008
- KNUD KEAGBALLE : "Treatment of psoriasis by the topical application of the novel cholecalciferol analogue calcipotriol (MC 903)", ARCHIVES OF DERMATOLOGY, vol. 125, no. 12, 31 December 1989 (1989-12-31), pages 1647 - 1652, XP009027910, ISSN: 0003-987X, DOI: 10.1001/archderm.1989.01670240047011
- DUBERTRET L; WALLACH D; SOUTEYRAND P; PERUSSEL M; KALIS B; MEYNADIER J; CHEVRANT-BRETON J; BEYLOT C; BAZEX J A; JURGENSEN H J: "Efficacy and safety of calcipotriol ( MC 903) ointment in psoriasis vulgaris", JOURNAL OF THE AMERICAN ACADEMY OF DERMATOLOGY, vol. 27, no. 6, 31 December 1992 (1992-12-31), pages 983 - 988, XP023982714, ISSN: 0190-9622, DOI: :10.1016/0190-9622(92)70299-U

## Description

This application claims the benefit of United States Provisional Application No. 62/639,742 filed on March 7, 2018.

### Field of the Invention

The field of the invention is combination therapies for treating hyperproliferative skin disorders.

### Background

The background description includes information that may be useful in understanding the present invention. It is not an admission that any of the information provided herein is prior art or relevant to the presently claimed invention, or that any publication specifically or implicitly referenced is prior art.

Cell proliferation disorders of the skin such as psoriasis, actinic keratosis, and non melanoma skin cancers (e.g., basal cell carcinoma and squamous cell carcinoma) are common, but treatments often lack the desired effectiveness, have inconvenient dosing regimens, and/or have severe side effects.

Current pharmaceutical approaches to treating skin proliferation disorders (such as psoriasis) include the use of corticosteroids, vitamin D analogs, and retinoids. In some instances phototherapy is used to treat skin proliferation disorders, and can be used in combination with pharmaceutical interventions. Phototherapy typically involves exposure to UVB and/or UVA light, and can include treatment with photosensitizers (e.g. using psoralen in combination with exposure to UVA light).

More recently, biologics have been used to treat skin proliferative disorders. These are typically monoclonal antibodies or antibody fragments that target specific components of the immune system or cytokines involved in inflammation. Examples of such biologics include Humira^{™}, Amevive^{™}, ABT-874, Enbrel^{™}, Remicade^{™}, and Stelara^{™}. Unfortunately such biologics are and expensive, and the use of such biologics is associated with increased incidence of cancer and certain types of infection.

Actinic keratoses are common pre-cancerous skin lesions that show excessive proliferation of skin cells, and are the third most common reason for consulting a dermatologist. These can be treated by excision, freezing, and/or the application of pharmaceutical agents to the lesion. Drugs used to treat these lesions include 5-fluorouracil (5-FU), diclofenac, ingenol, and imiquimod, Unfortunately, side effects (which include discomfort, redness, and blistering) and/or inconvenient dosing regimens limit patient compliance, and therefore, the efficacy of these approaches.

Calcipotriol is an FDA-approved topical medication for treatment of skin hyperproliferative disorders, and is hypothesized to act via induction of thymic stromal lymphopoietin ("TSLP") expression. The immune effects of TSLP expression have been shown to include antitumor immunity in barrier-defective skin (see Trevor J. Cunningham et al., Randomized trial of calcipotriol combined with 5-fluorouracil for skin cancer precursor immunotherapy, 127(1) J. Clinical Investigation 106-16 (2017)). Where a definition or use of a term in a reference is inconsistent or contrary to the definition of that term provided herein, the definition of that term provided herein applies and the definition of that term in the reference does not apply.

Calcipotriol has been used in combination with other drugs. For example, treatment with calcipotriol ointment combined with 5-fluorouracil cream can decrease the number of actinic keratoses compared to treatment with 5-fluorouracil alone. The combined use of calcipotriol ointment and 5-fluorouracil cream on face, scalp, or upper extremities has been found to induce TSLP, HLA class II, and natural killer cell group 2D ligand expression in keratinocytes of these lesions. Calcipotriol and 5- fluorouracil are thought to activate a CD4+ T cell-mediated immunity against actinic keratosis. However, this treatment regiment maintains the limitations of 5- fluorouracil, including side effects that limit patient compliance.

Thus, there is still a need for treatments for cell hyperproliferation disorders that are both effective and well tolerated.

INDIAN DERMATOLOGY ONLINE JOURNAL, 2017, vol. 8, no. 4, 235-245 describes the use of topical corticosteroids, Vitamin D analogs, salicylic acid, coal tar, and anthralin in treating psoriasis.

DERMATOLOGIC CLINICS, 2015, vol. 33, no. 1, 73-77 describes the use of vitamin D derivatives, corticosteroids and retinoids in treating psoriasis.

Kinex Pharmaceuticals: "Kinex Pharmceuticals announces allowance of the Investigational New Drug (IND) application for KX2-391 ointment by the United States Food and Drug Administration (FDA)",, 24 July 2014 (2014-07-24), pages 1-3 describes the allowance by the US FDA for KX2-391 Ointment for the commencement of a Phase I clinical study in actinic keratosis.

American Journal of Clinical Dermatology, 2012, vol. 2, no. 2, 95-120 describes the clinical effect of oral vitamin D2 supplementation on psoriasis.

Archives of Dermatology, 1989, vol. 125, no. 12, 1647-1652 describes the treatment of psoriasis by the topical application of calcipotriol.

Journal of the American Academy of Dermatology, 1992, vol. 27, no. 6, 983-988 describes the efficacy and safety of calcipotriol ointment in psoriasis vulgaris.

### Summary of The Invention

The present invention is set out in the appended claims.

A first aspect of the present invention is a pharmaceutical composition for use in the treatment of a hyperproliferative skin disorder, comprising a first compound, wherein the first compound is KX01 and has the formula: 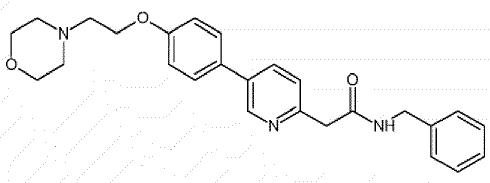 and a second compound comprising calcipotriol.

A second aspect of the present invention is a topical preparation for use in combination with exposure to a blue light in treating a proliferative skin condition, wherein the topical preparation comprises a first compound, wherein the first compound is KX01, and the blue light comprises a wavelength from 280nm to 500 nm, wherein the topical preparation includes the first compound in a first amount that provides a first reduction in proliferation, wherein the blue light is selected to provide a second reduction in proliferation, wherein use of the topical preparation in combination with the blue light provides a combined reduction in proliferation that is greater than the sum of a first reduction in proliferation and the second reduction in proliferation, wherein the proliferative skin condition is actinic keratosis or psoriasis.

Thus, one embodiment of the present invention is a pharmaceutical composition for use in the treatment of a hyperproliferative skin disorder that includes a first compound, wherein the first compound is KX01, having an SRC-kinase inhibition activity, an inhibition of tubulin polymerization activity, a cell cycle arrest at G2/M activity, and/or a induction of apoptosis activity, and a second compound comprising calcipotriol which is a vitamin D analog, where the first compound and the second compound are present in amounts sufficient to treat a hyperproliferative disorder in an affected area. Such composition can be formulated for topical application, for example as a foam, a cream, a paste, an ointment, a gel, a solution, a liquid suspension, a droplet suspension, an aerosol, and/or a powder. The vitamin D analog can also have a G0/G1 arrest activity, an anti-proliferation activity, an anti-inflammation activity, an induction of keratinocyte differentiation activity, an activity that decreases the number of pro-inflammatory T-cells, an apoptosis inducing activity.

The first compound is KX01, which has the structure shown in Formula 2.

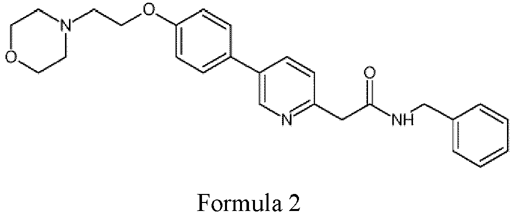

In such a composition the second compound is a vitamin D3 analog, calcipotriol (calipotriene). The composition can be formulated to provide a molar ratio of the first compound to the second compound of from 160:3 to 5:243 at the affected area. In some embodiments the composition this molar ratio is selected to provide an effect at the affected area that exceeds the additive effect of the first compound and the second compound when applied individually at corresponding concentrations. In some embodiments composition also includes a retinoid.

The references to the methods of treatment by therapy in this description are to be interpreted as references to compounds, pharmaceutical compositions and medicaments of the present invention for use in those methods. The pharmaceutical composition of the present invention as described herein can be used in a method of treating a skin condition (such as actinic keratosis or psoriasis) by providing a topical preparation (e.g. a foam, a cream, a paste, an ointment, a gel, a solution, a liquid suspension, a droplet suspension, an aerosol, and/or a powder). The molar ratio molar ratio of the first compound to the second compound can range from 160:3 to 5:243 or from 1:1 to 1:2,500 at the affected area. The first compound is KX01 (as described above). The second compound is calcipotriol (calcipotriene). In some embodiments the topical composition also includes a retinoid. The method also includes a step of applying the topical preparation to an area affected by the skin condition on a schedule that is effective to treat the skin condition (e.g. from once a week to four times a day). Skin conditions that can be treated in this fashion include actinic keratosis and psoriasis. The vitamin D analog can have a G0/G1 arresting activity, an anti-proliferation activity, an anti-inflammatory activity, an activity that induces keratinocyte differentiation, an activity that decreases the number of pro-inflammatory T-cells, and/or an activity that induces apoptosis. The retinoid can have an activity that induces keratinocytes proliferation, modulates epidermal differentiation, stimulates extracellular matrix production, and/or alters sebocyte differentiation. In a preferred embodiment the first topical composition includes the first compound in a first amount and the second topical composition comprises the second compound in a second amount, where the first amount and the second amount are selected to provide a combined therapeutic effect. This combined therapeutic effect is greater than the sum of the therapeutic effects provided by application of the first compound at the first amount and by application of the second compound at the second amount.

Also described is a kit for treating a skin cell hyperproliferation disorder, having a first topical composition that includes a first compound (KX01, as shown above) with two or more of a SRC-kinase inhibiting activity, a tubulin polymerization inhibiting activity, an activity that arrests the cell cycle at G2/M, and an activity that induces apoptosis, along with a second topical composition that includes a second compound calcipotriol and may also include a vitamin D3 analog. In some embodiments the kit includes both a vitamin D analog and a retinoid. The first compound and the second compound are present in their respective topical compositions in amounts sufficient to provide a reduction or elimination of the skin cell hyperproliferation disorder when the first and second topical compositions are applied individually according to schedule. The molar ratio molar ratio of the first compound to the second compound can range from 160:3 to 5:243 or from 1:1 to 1:2,500. Such topical compositions can be provided as a foam, a cream, a paste, an ointment, a gel, a solution, a liquid suspension, a droplet suspension, an aerosol, and/or a powder. In a preferred embodiment the first topical composition includes the first compound in a first amount and the second topical composition comprises the second compound in a second amount, where the first amount and the second amount are selected to provide a combined therapeutic effect. This combined therapeutic effect is greater than the sum of the therapeutic effects provided by application of the first compound at the first amount and by application of the second compound at the second amount.

Another embodiment of the present invention is a topical preparation for use in a method of treating a proliferative condition (actinic keratosis and/or psoriasis), wherein the topical preparation includes a first compound, where the first compound is KX01, as shown above, has two or more of a SRC-kinase inhibiting activity, a tubulin polymerization inhibiting activity, an activity that arrests the cell cycle at G2/M, and an activity that induces apoptosis, and the method includes applying the topical preparation to an area affected by the skin condition on a schedule effective to treat the skin condition, in combination with directing a blue light having a wavelength from 280 nm to 500 nm, which can be provided as either a narrow or broad band emission, onto the area affected by the skin condition at a specified light flux and for a specified exposure time. Suitable light sources include LED/LCD and fluorescent lights, and can emit visible blue light (e.g. 311 nm to 460 nm), UVA, and/or UVB). The topical preparation can be formulated as formulated as a foam, a cream, a paste, an ointment, a gel, a solution, a liquid suspension, a droplet suspension, an aerosol, and/or a powder. In some embodiments the topical preparation further includes a vitamin D analog (e.g. a vitamin D3 analog and/or calcipotriol) and/or a retinoid (e.g. an acetylenic retinoid and/or tazarotene). In such embodiments the retinoid can have an activity that induces keratinocytes to proliferate, an activity that modulates epidermal differentiation, an activity that stimulates extracellular matrix production, and/or an activity that alters of sebocyte differentiation. In some embodiments the topical preparation includes the first compound (and second compound, if present) in an amount(s) that provides a first therapeutic effect and the specified light flux and exposure time are provided that can produce a second therapeutic effect, however the method provides a third therapeutic effect that is greater than the sum of a first therapeutic effect and the second therapeutic effect.

Various objects, features, aspects and advantages of the inventive subject matter will become more apparent from the following detailed description of preferred embodiments, along with the accompanying drawing figures in which like numerals represent like components.

### Brief Description of The Drawings

FIG. 1: Results of an experiment comparing cell growth in response to KX01, Calcipotriol, and KX01 + calcipotriol at a 1:5 ratio.
FIG. 2: Results of an experiment comparing cell growth in response to KX01, Calcipotriol, and KX01 + calcipotriol at a 1:2 ratio.
FIG. 3: Results from two experiments comparing cell growth in response to KX01, Calcipotriol, and KX01 + calcipotriol at a 2:1 ratio.
FIG. 4: Inhibition of human keratinocyte growth versus KX01 concentration (treatment duration: 72 hours; cell growth measured using the MTT assay). The IC₅₀ of KX01 = 41.9 nM.
FIG.5: Inhibition of human keratinocyte growth versus calcipotriol concentration (treatment duration: 72 hours; cell growth measured using the MTT assay). The IC₅₀ of calcipotriol = 95.5 nM.
FIG. 6: Dose response surface of cell viability versus calcipotriol concentration versus KX01 concentration. Synergistic combinations of calcipotriol and KX01 are below the surface of additivity (indicating enhanced inhibition of growth), and antagonistic combinations are above the surface of additivity (indicative of reduced inhibition of growth).
FIG. 7: Inhibition of CCD1106 KERTr keratinocyte growth by tazarotene, as determined by staining - not according to the invention.
FIG. 8: Inhibition of A431 cell growth by tazarotenee, as determined by staining - not according to the invention.
FIG. 9: Results of exposure of keratinocytes to a light source providing primarily visible blue light (top left) or to a light source providing primarily UVB light (top right).
FIG. 10: Improved reduction in viability of keratinocytes at increased blue light intensity.
FIG. 11: Effects of blue light exposure in combination with either KX01 or calcipotriol from 0 hours (no blue light exposure) to 6 hours on keratinocyte proliferation.
FIG. 12: PSI values determined for combined effects of treatment with blue light, KX01, and/or calcipotriol on skin cell proliferation.

### Detailed Description

The described subject matter provides compositions and methods in which a vitamin D analog synergistically enhances the antiproliferative effects of an antiproliferative compound for the treatment of cell hyperproliferation disorders, particularly dermatological disorders characterized by increased proliferation of skin cells (e.g. psoriasis, actinic keratosis, etc.).

Within the context of this application the term Imax refers to the maximal inhibitory concentration of a compound or therapy, the term IC50 refers to a value at which 50% of the maximum inhibitory effect realized is achieved, and the term H is related to the steepness of the slope of a calculated curve or surface derived from a mathematical model.

Various objects, features, aspects and advantages of the inventive subject matter will become more apparent from the following detailed description of preferred embodiments, along with the accompanying drawing figures.

The present invention includes pharmaceutical compositions that include a first compound which is KX01 that exhibits at least two of: SRC-kinase inhibition, inhibition of tubulin polymerization, cell cycle arrest at G2/M, and induction of apoptosis. The pharmaceutical composition further includes a second compound that comprises calcipotriol and may further comprise a retinoid (i.e. a retinoid or retinoid analog). The first and second compounds are present in the pharmaceutical composition in amounts sufficient to treat a cell hyperproliferation disorder, such actinic keratosis or psoriasis. In preferred embodiments the first and second compounds are present in amounts that provide a synergistic effect on inhibition of proliferation/growth of skin cells relative to their individual contributions. In some embodiments such drug combinations can be used in combination with controlled exposure to light, for example blue light (e.g. 280nm to 500 nm light), UV/A light, and/or UV/B light.

The recitation of ranges of values herein is merely intended to serve as a shorthand method of referring individually to each separate value falling within the range. Unless otherwise indicated herein, each individual value is incorporated into the specification as if it were individually recited herein.

In some embodiments, the numbers expressing quantities of ingredients, properties such as concentration, reaction conditions, and so forth, used to describe and claim certain embodiments of the invention are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the invention are approximations, the numerical values set forth in the specific examples are reported as precisely as practicable. The numerical values presented in some embodiments of the invention may contain certain errors necessarily resulting from the standard deviation found in their respective testing measurements.

Unless the context dictates the contrary, all ranges set forth herein should be interpreted as being inclusive of their endpoints and open-ended ranges should be interpreted to include only commercially practical values. Similarly, all lists of values should be considered as inclusive of intermediate values unless the context indicates the contrary.

As used in the description herein and throughout the claims that follow, the meaning of "a," "an," and "the" includes plural reference unless the context clearly dictates otherwise. Also, as used in the description herein, the meaning of "in" includes "in" and "on" unless the context clearly dictates otherwise.

Groupings of alternative elements or embodiments of the invention disclosed herein are not to be construed as limitations. Each group member can be referred to and claimed individually or in any combination with other members of the group or other elements found herein. One or more members of a group can be included in, or deleted from, a group for reasons of convenience and/or patentability. When any such inclusion or deletion occurs, the specification is herein deemed to contain the group as modified thus fulfilling the written description of all Markush groups used in the appended claims.

The first compound is KX01 (N-benzyl-2-(5-{4-[2-(morpholine-4-yl)ethoxy]phenyl}pyridine-2-yl)acetamide. The chemical structure of KX01 is represented by Formula 2: 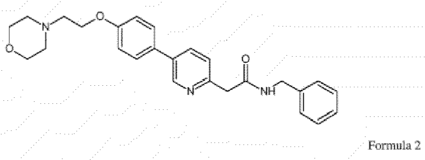 KX01 has been shown to inhibit SRC-kinase, inhibits tubulin polymerization, and induces apoptosis by G2/M arrest.

Calcipotriol is a vitamin D3 analog that has been observed to induce apoptosis in a variety of cell types. Without wishing to be bound by a particular theory, the inventors hypothesize that the combination of an antiproliferative compound with a vitamin D analog is beneficial, because they have different mechanisms of action that target two different phases of the cell cycle.

Surprisingly, the inventors identified synergistic (e.g., more than additive) effects in regards to inhibition of skin cell proliferation/growth when such compounds are used together. Advantageously, combining KX01 and calcipotriol expands the clinical utility of KX01 ointment to the treatment of hyperproliferative skin disorders, while synergistic effects can provide for increased effectiveness and/or reduced dosages.

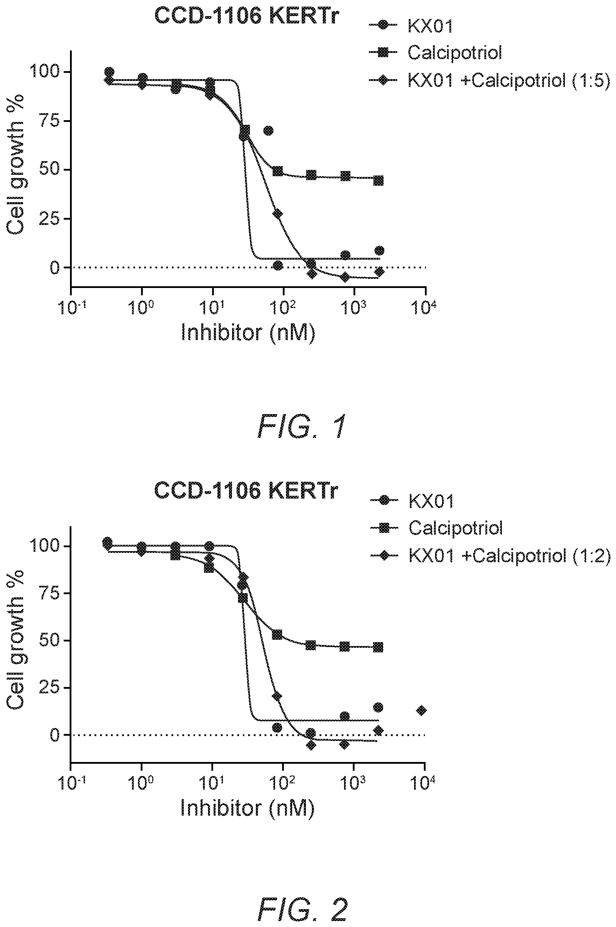

Typical data from studies of the effects of compound KX01 provided in combination with calcipotriol on growth of human keratinocytes in culture (cell line CCD- 1106 KERTr) are shown in FIGs. 1 to 3 and Tables 1 to 6. FIG. 1 and Tables 1 and 2 show the degree of growth inhibition found in a typical study performed with KX01 and calcipotriol at a 1:5 ratio.

**Table 1**

| **nM** | **Log** | **KX01** | **Calcipotriol** | **KX0l+Calcipotriol (1:5)** |
|---|---|---|---|---|
| **0.333** | -0.47756 | 100.45 | | 96.21622 |
| **1** | 0 | 97.0297 | | 93.33333 |
| **3** | 0.477121 | 90.90909 | 94.0367 | 92.61261 |
| **9** | 0.954243 | 94.68947 | 89.90826 | 88.01802 |
| **27** | 1.431364 | 67.14671 | 70.45872 | 70.27027 |
| **81** | 1.908485 | 1.350135 | 49.3578 | 27.74775 |
| **243** | 2.385606 | 2.070207 | 47.33945 | -2.79279 |
| **729** | 2.862728 | 6.660666 | 46.97248 | -4.32432 |
| **2187** | 3.339849 | 8.910891 | 44.77064 | -1.71171 |
| **6561** | 3.81697 | | 19.17431 | |
| **19683** | 4.294091 | | -9.3578 | |

Table 1 shows the results of cell viability studies comparing cell growth in response to KX01, calcipotriol, and KX01 + calcipotriol at a 1:5 ratio, as graphically depicted in FIG. 1A. Table 2 shows typical GI₅₀ and combination index (CI) values for KX01 and calcipotriol (1:5) derived from such studies.

**Table 2**

| | **GI₅₀** | **CI (Combination Index)** |
|---|---|---|
| **KX01** | 32 nM | |
| **Calcipotriol** | 265 nM | 0.37 |
| **KX0l+Calcipotriol (1:5)** | 43 nM (7.2 nM+35.8 nM) | |

FIG. 2 shows typical results of similar studies in which KX01 and calcipotriol are used in a 1:2 ratio. Typical numerical data from such a study is shown in Table 3. Table 4 shows typical GI50 and combination index (CI) values for KX01 and calcipotriol (1:2) derived from such studies.

**Table 3**

| **nM** | **Log** | **KX01** | **Calcipotriol** | **KX0l+Calcipotriol (1:5)** |
|---|---|---|---|---|
| **0.333** | -0.47756 | 102.3 | | 100.7 |
| **1** | 0 | 99.9 | | 97.2 |
| **3** | 0.477121 | 100.0 | 95.3 | 95.7 |
| **9** | 0.954243 | 100.2 | 88.8 | 93.8 |
| **27** | 1.431364 | 79.8 | 72.5 | 83.6 |
| **81** | 1.908485 | 4.1 | 53.2 | 20.8 |
| **243** | 2.385606 | 1.2 | 47.7 | -5.4 |
| **729** | 2.862728 | 9.9 | 47.1 | -4.8 |
| **2187** | 3.339849 | 14.9 | 46.7 | 2.5 |
| **6561** | 3.81697 | | 25.2 | |
| **19683** | 4.294091 | | -13.4 | |

**Table 4**

| | **GI₅₀** | **CI (Combination Index)** |
|---|---|---|
| **KX01** | 38 nM | |
| **Calcipotriol** | 310 nM | 0.54 |
| **KX0l+Calcipotriol (1:5)** | 49 nM (16.3 nM+32.7 nM) | |

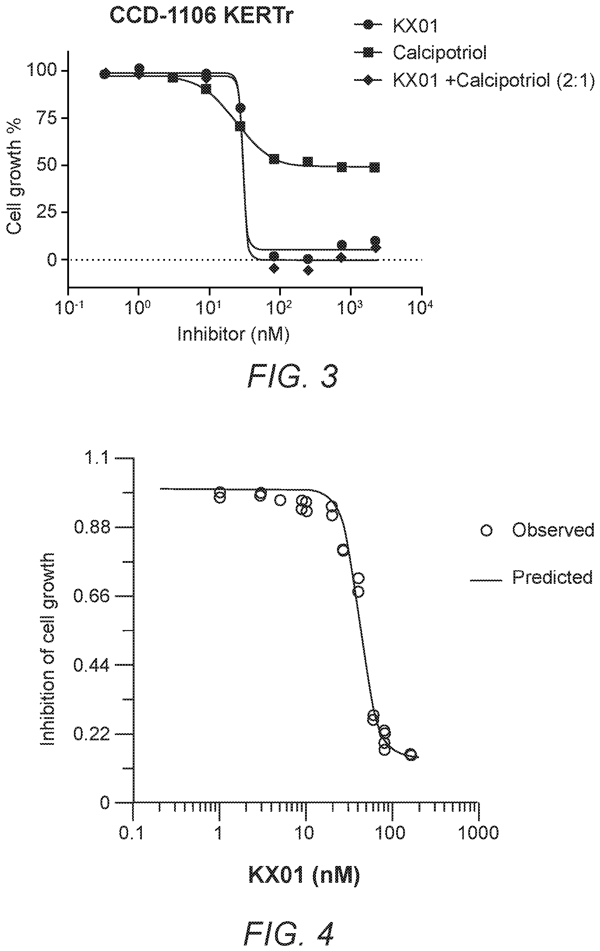

FIG. 3 shows typical results of studies in which KX01 and calcipotriol are used in a 2:1 ratio. Typical numerical data from such a study is shown in Table 5. Table 6 shows typical GI₅₀ and combination index (CI) values for KX01 and calcipotriol (1:5) derived from such studies.

**Table 5**

| **nM** | **Log** | **KX01** | **Calcipotriol** | **KX0l+Calcipotriol (1:5)** |
|---|---|---|---|---|
| **0.333** | -0.47756 | 98.0 | | 98.8 |
| **1** | 0 | 101.3 | | 97.8 |
| **3** | 0.477121 | 96.6 | 96.1 | 96.0 |
| **9** | 0.954243 | 98.2 | 89.7 | 95.3 |
| **27** | 1.431364 | 80.1 | 70.1 | 80.2 |
| **81** | 1.908485 | 1.6 | 52.8 | -4.6 |
| **243** | 2.385606 | -0.1 | 51.0 | -5.9 |
| **729** | 2.862728 | 7.3 | 48.7 | 0.8 |
| **2187** | 3.339849 | 9.5 | 48.3 | 5.8 |
| **6561** | 3.81697 | | 26.5 | |
| **19683** | 4.294091 | | -15.7 | |

**Table 6**

| | **GI₅₀** | **CI (Combination Index)** |
|---|---|---|
| **KX01** | 36 nM | |
| **Calcipotriol** | 339 nM | 0.59 |
| **KX0l+Calcipotriol (1:5)** | 30 nM (20nM+10nM) | |

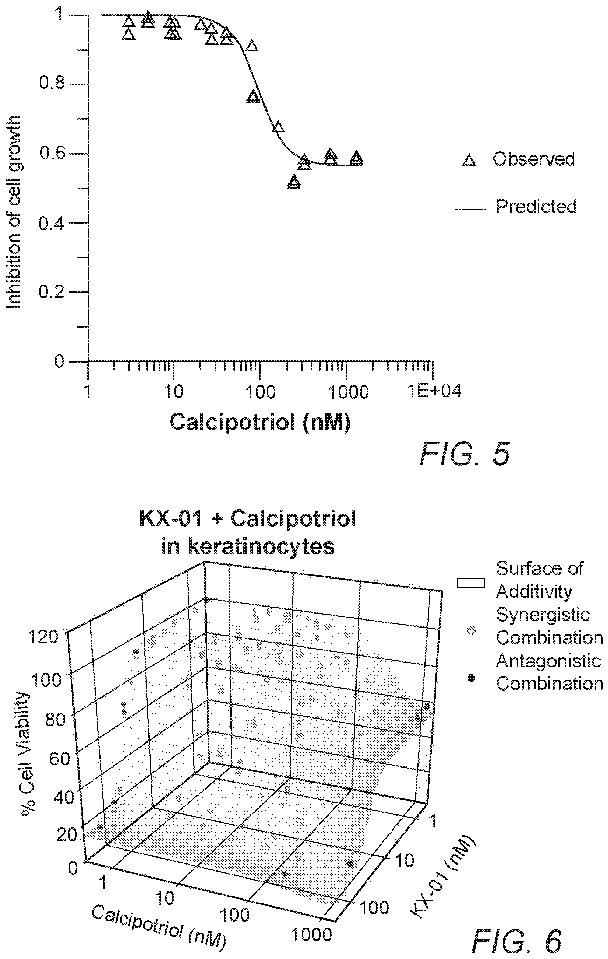

In order to determine the range of concentrations over which compounds such as KX01 and calciptriol can inhibit keratinocyte growth and subsequent combinatorial effects, individual dose response curves were determined using a 72 hour exposure to either KX01 or calcipotriol in order to determine individual IC₅₀ values. Results are shown in FIG. 4 (which shows typical results for KX01) and FIG. 5 (which shows typical results for calcipotriol). A human keratinocyte cell line was used in these studies. The IC₅₀ for KX01 was determined to be 41.9 nM; the IC₅₀ for calcipotriol was determined to be 95.5 nM.

Individual dose response curves for KX01 and calcipotriol as determined above can be used to generate dose response surfaces representative of the additive effects of these compounds on keratinocyte growth. Such dose response surfaces can be utilized in detailed combinatorial studies in order to determine if the effects such compounds in combination is antagonistic, merely additive, or showed positive synergistic effects. Synergistic effects can be expressed as "PSI" or Ψ, which has been used as a measure of synergy (see Ariens EJ, Van Rossum JM and Simonis AM, Affinity, intrinsic activity and drug interactions, Pharmacol Rev. 1957 Jun. 9(2): 218-36; Chakraborty A and Jusko WJ, Pharmacodynamic interaction of recombinant human interleukin-10 and prednisolone using in vitro whole blood lymphocyte proliferation, J. Pharm. Sci. 2002, 91: 1334). A PSI value of 1 corresponds to an additive effect. A PSI value of less than 1 indicates a synergistic effect, and a PSI value of greater than 1 indicates an antagonistic effect.

Cell viability was measured in a number of experiments. In Experiment 1, the concentrations of calcipotriol were: 0, 3, 9, 27, 81, and 243 nM, and the concentrations of KX01 were: 0, 5, 10, 20, 40, 60, 80, and 160 nM. In Experiment 2, the concentrations of calcipotriol were: 0, 5, 10, 20, 40, 80, 160, 320, and 1280 nM, and the concentrations of KX01 were: 1, 3, 9, 27, and 81 nM. The results are shown in FIG. 6, which shows the calculated additive response surface derived from individual dose response curves for each compound and individual data points representing the results of individual combinations. Synergistic effects are indicated when the observed response lies below the additive response surface; antagonistic effects are indicated when the observed response lies above the additive response surface. It is notable that unexpected synergy is observed across a range of concentrations and combinations.

Tables 7, 8, and 9 show the results for determination of individual IC₅₀ and maximum inhibitory concentration values for KX01 and calcipotriol, as well as the PSI value for the drug combination. Table 7 shows values derived from the study depicted in FIG. 6. Tables 8 and 9 show values derived from subsequent replicate studies.

**Table 7**

| **Parameters** | **Estimate (CV%)** |
|---|---|
| **PSI** | 0.571 (3.72) |
| **Max Inhibition KX01** | 0.856 (1.31) |
| **IC₅₀ KX01** | 41.3 (3.62) |
| **Max Inhibition Calcipotriol** | 0.474 (2.74) |
| **IC₅₀ Calcipotriol** | 112 (7.01) |

**Table 8**

| **Parameters** | **Estimate (CV%)** |
|---|---|
| **PSI** | 0.626 (3.26) |
| **Max Inhibition KX01** | 0.77 (2.11) |
| **IC₅₀ KX01** | 32.8 (6.29) |
| **Max Inhibition Calcipotriol** | 0.36 (3.00) |
| **IC₅₀ Calcipotriol** | 59.3 (10.4) |

**Table 9**

| **Parameters** | **Estimate (CV%)** |
|---|---|
| **PSI** | 0.787 (4.99) |
| **Max Inhibition KX01** | 0.741 (5.21) |
| **IC₅₀ KX01** | 47.0 (14.8) |
| **Max Inhibition Calcipotriol** | 0.439 (4.99) |
| **IC₅₀ Calcipotriol** | 112 (5.72) |

As shown above, a significant synergistic effect in inhibition of keratinocyte growth is consistently found when KX01 and calcipotriol are used in combination. In some embodiments such synergy is observed when the concentration of KX01 is about 1 to about 30 nM and the concentration of calcipotriol is about 3 to about 300 nM, and is present when KX01 and calcipotriol are provided in a suitable formulation in amounts sufficient to generate such concentrations in the tissue to be treated. Such formulation can provide KX01 and calcipotriol in amounts that provide a ratio of these active compounds that in turn provides a synergistic effect in regards to inhibition of keratinocyte growth.

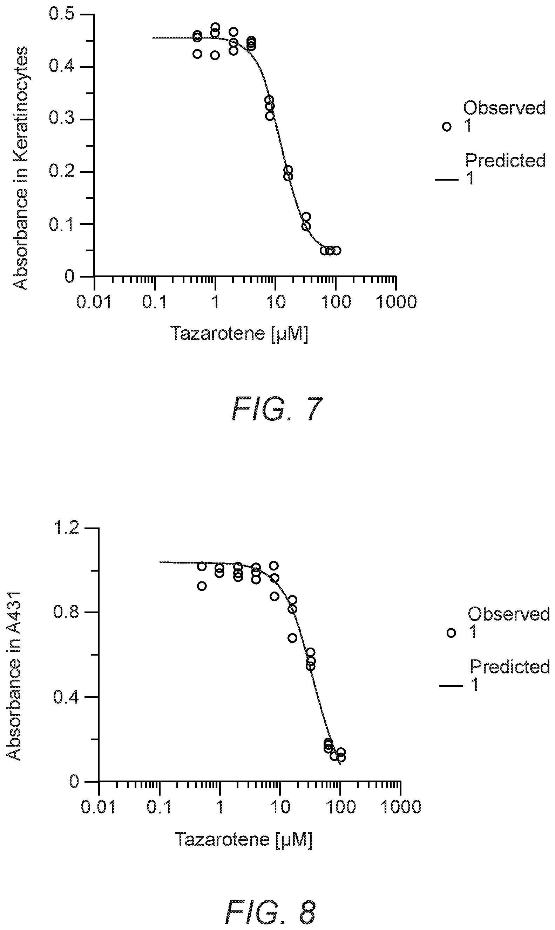

Inventors have also found that treatment with KX01 can be combined with a retinoid and/or retinoid analog to provide a synergistic (i.e. greater than additive) effect in the inhibition of cell proliferation/growth that is characteristic of hyperproliferative disorders. This disclosure does not form part of the invention as claimed. Examples of suitable retinoids include retinol, retinal, retinoic acid isotretinoin, alitreninoin, etretinate, acitretin, adapalene, bexarotene, and/or tazarotene. FIGs. 7 and 8 show the effects of tazarotene on growth of cells in culture, including CCD-1106 KERTr keratinocytes (FIG. 7) and A431 cells (epidermoid cells obtained from a human squamous cell carcinoma, FIG. 8). Numerical data from these studies is provided in Table 10, which shows data derived from CCD- 1106 KERTr keratinocytes and from A431 cells.

**Table 10**

| **Parameter** | **Estimate (CV%) (Keratinocytes)** | **Estimate (CV%) (A431)** |
|---|---|---|
| **R0** | 0.455 (1.07) | 1.03 (1.94) |
| **Maximum inhibition** | 0.903 (1.68) | 1.09(12.3) |
| **IC₅₀** | 12.4 (4.12) | 36.3 (19.8) |
| **H** | 2.11 (7.93) | 1.66 (19.9) |

Studies on the effects of combined treatment with KX01 and tazarotene, not according to the invention, were performed, and showed a clear and unexpected synergistic effect. Typical results are shown in Table 11.

**Table 11**

| | **KX01 (nM)** | **Tazarotene (nM)** |
|---|---|---|
| **IC₅₀** | 45 | 13.3 µM |
| **Imax** | 0.92 | 1.1 |
| **H** | 3.3 | 1.0 |
| | | |

| | **PSI value** | **95% Confidence Interval** |
|---|---|---|
| | 0.76 (3.3%CV) | 0.7-0.8 |

In addition to growth inhibition, Inventors also investigated the effect of KX01 on apoptosis in keratinocytes. CCD-l 106 KERTr cells (lx10⁶) were seeded in 60 mm dishes and treated with test compounds at varying concentrations (e.g. KX01: 20 to 500nM; calcipotriol 50 to 1000nM) for 24 hours. Cell apoptosis was assessed by using a FITC annexin V apoptosis detection kit (BD Biosciences^{™}) according to manufacturer's instruction. Briefly, cells were collected in lx binding buffer and incubated with fluorescein isothiocyanate (FITC)-conjugated annexin V and propidium iodide (PI) for 15 minutes at room temperature in the dark, followed by flow cytometry analysis using a LSRFortessa^{™} flow cytometer (BD Biosciences^{™}). Results are summarized in Table 12, which shows the percentage of late apoptic cells identified for various KX01 and calcipitriol combinations.

**Table 12**

| **[Calcipotriol], nM** | | | | | | |
|---|---|---|---|---|---|---|
| **1,000** | 4.19 | 3.15 | 4.61 | 8.74 | 4.64 | |
| **500** | 4.13 | 2.71 | 4.88 | 7.93 | 5.89 | |
| **200** | 2.06 | 2.55 | 4.97 | 7.94 | 6.13 | |
| **50** | 2.41 | 2.12 | 4.50 | 5.70 | 5.36 | |
| **0** | 2.08 | 2.09 | 3.50 | 6.33 | 4.34 | |
| | **0** | **20** | **40** | **100** | **500** | **[KX01], nM** |

Synergy between KX01 and calcipotriol in regards to increasing the number of keratinocytes undergoing apoptosis is evident. For example the effect of 200 nm calcipotriol is not distinguishable from that of control cells receiving no drug treatment, however the same concentration of calcipotriol enhances the effect of KX01 by approximately 40%.

The inventive subject matter also includes using blue light at a wavelength from 280 nm to 500 nm, and my further include using UV/A light, and/or UV/B light, to illuminate skin during, prior to, or following treatment with KX01 (either alone or in combination with other compounds), particularly where synergistic effects permit reduction in blue light exposure and/or drug dosage relative to either light exposure or chemotherapy alone. Any suitable source of blue light can be used. Suitable sources of blue light include incandescent bulbs, fluorescent lights, LED light sources, and laser light sources. Such a light source can be a broad spectrum light sources (i.e. providing a range of wavelengths exceeding 20 nm in width) or a narrow spectrum light source (i.e. providing a range of wavelengths spanning 20 nm or less). In some embodiments the light source can produce primarily (i.e. >90%) blue light. In other embodiments the light source can produce both blue, and non-blue light, with non-blue light selectively blocked by an optical filter. In a preferred embodiment the light source for visible blue light is a panel or fluorescent light emitting a narrow bandwidth light centered at 460nm. In another preferred embodiment the light sources for UVB light is a fluorescent and/or LED/LCD panel light emitting a narrow bandwidth centered at 311 nm.

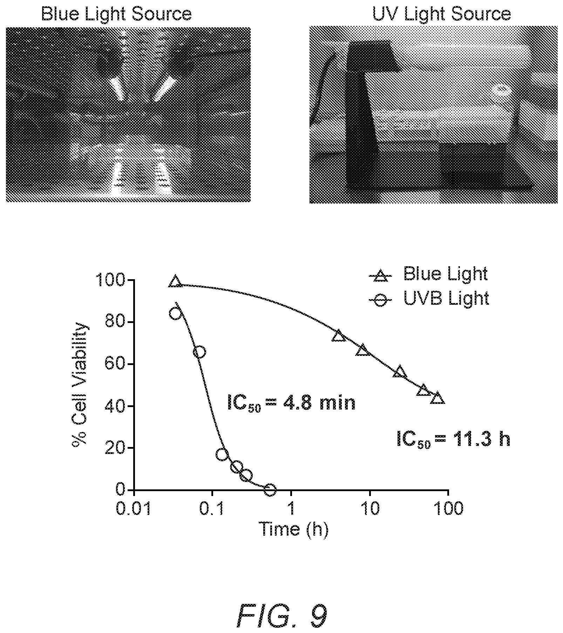

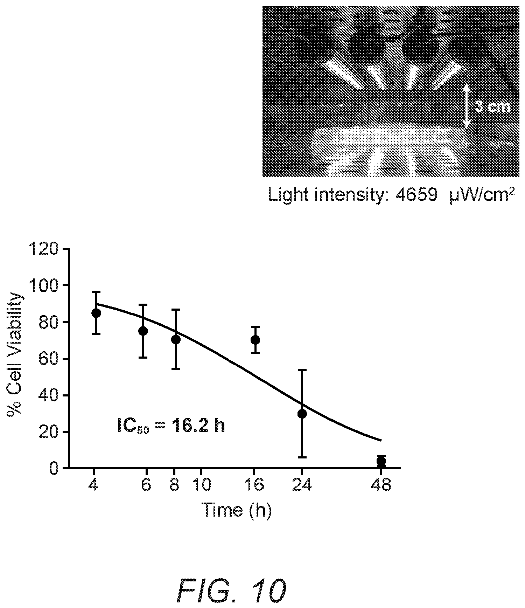

It should be appreciated that exposure to blue light alone can reduce cell viability, and that different sources of blue light can provide different effects. An example of this is provided in FIG. 9, which shows the results of exposure of CCD 1106 KERTr human keratinocytes cells to different blue light sources (i.e. blue visible light or UVB) for 72 hours. It is apparent that UVB light is significantly more effective in reducing keratinocyte viability relative to visible blue light, although both are effective. As shown in FIG. 10, the overall effectiveness (in terms of absolute reduction in cell viability) of light sources providing primarily visible blue light can be improved by increasing its intensity.

The effects of combined treatment with KX01, calcipotriol and/or blue/UV light exposure were characterized using a cell proliferation assay. Cell proliferation was assessed by using an MTT assay. Cells were seeded in 96-well plate (3,000-5,000 cells per well) and treated with test compounds at varying concentrations (e.g. KX01: 5 to 80 nM; calcipotriol 5 to 1,280 nM) for 72 hours, either in the dark or exposed to blue light irradiation (for 2, 4, or 6 hours). To each well, 10 pl of 3-(4,5-dimethylthiazol-2-yl)-2,5-diphenyltetrazolium bromide (MTT) (RPI Corporation^{™}) solution (5 mg/ml in PBS) was added and incubated at 37 °C for 3 hours. Next, 100 µl of 20% SDS was added to each well. After overnight incubation at 37 °C, the absorbance in each well was measured at 570 nm in a microplate reader (Molecular Devices^{™}). The cell growth curve and GI₅₀s were calculate using GraphPad Prism^{™} 5 software.

Tables 13 and 14 show the results of cell growth inhibition studies (expressed as a percentage relative to control cell growth) for treatment with either KX01 or calcipotriol in combination with exposure to blue light.

**Table 13**

| **Blue light (hours)** | | | | | | |
|---|---|---|---|---|---|---|
| **6** | 50 | 56 | 70 | 88 | 87 | |
| **4** | 41 | 47 | 64 | 87 | 92 | |
| **2** | 26 | 27 | 63 | 92 | 97 | |
| **0** | 0 | 4 | 40 | 90 | 96 | |
| | **0** | **10** | **20** | **40** | **80** | **[KX01], nM** |

Table 13 shows the results of a cell proliferation assay performed using KX01 and blue light and the CCD 1106 KERTr human keratinocyte cell line. The percentage of growth inhibition relative to untreated control is shown.

**Table 14**

| **Blue light (hours)** | | | | | | | |
|---|---|---|---|---|---|---|---|
| **6** | 53 | 56 | 59 | 71 | 72 | 66 | |
| **4** | 48 | 53 | 58 | 70 | 71 | 69 | |
| **2** | 25 | 32 | 39 | 55 | 66 | 62 | |
| **0** | 0 | 9 | 24 | 45 | 48 | 43 | |
| | **0** | **5** | **20** | **80** | **320** | **1,280** | **[calcipotriol], nM** |

Table 14 shows the results of a cell proliferation assay performed using calcipotriol and blue light and the CCD 1106 KERTr human keratinocyte cell line. The percentage of growth inhibition relative to untreated control is shown.

Tables 15 to 18 show the effects of combined treatment with KX01, calcipotriol, and exposure to blue light at 0 hours, 2 hours, 4 hours, and 6 hours of light exposure (respectively). Values are expressed as the percentage of growth inhibition relative to cells grown under control conditions (0 hours blue light, [KX01] = 0, [calcipotriol] = 0). It should be appreciated that synergy (i.e. greater than additive) effects between blue light exposure and combined KX01 and calcipotriol treatment are observed, for example at 2 hours of blue light exposure and combined treatment with 10 nM KX01 and 20 nM calcipotriol.

**Table 15**

| **[Calcipotriol], nM** | | | | | | |
|---|---|---|---|---|---|---|
| **1,280** | 43 | 60 | 86 | 95 | 98 | |
| **320** | 48 | 69 | 90 | 100 | 100 | |
| **80** | 45 | 67 | 89 | 100 | 100 | |
| **20** | 24 | 48 | 85 | 100 | 100 | |
| **5** | 9 | 22 | 80 | 100 | 100 | |
| **0** | 0 | 9 | 24 | 45 | 48 | |
| | **0** | **10** | **20** | **40** | **80** | **[KX01], nM** |

**Table 16**

| **[Calcipotriol], nM** | | | | | | |
|---|---|---|---|---|---|---|
| **1,280** | 62 | 79 | 91 | 95 | 100 | |
| **320** | 66 | 86 | 96 | 100 | 100 | |
| **80** | 55 | 84 | 96 | 100 | 100 | |
| **20** | 39 | 76 | 97 | 100 | 100 | |
| **5** | 32 | 47 | 92 | 100 | 100 | |
| **0** | 25 | 32 | 74 | 98 | 48 | |
| | **0** | **10** | **20** | **40** | **80** | **[KX01], nM** |

**Table 17**

| **[Calcipotriol], nM** | | | | | | |
|---|---|---|---|---|---|---|
| **1,280** | 69 | 81 | 91 | 96 | 97 | |
| **320** | 71 | 85 | 93 | 99 | 100 | |
| **80** | 70 | 83 | 90 | 100 | 100 | |
| **20** | 58 | 69 | 86 | 98 | 99 | |
| **5** | 53 | 62 | 78 | 94 | 94 | |
| **0** | 48 | 56 | 69 | 92 | 93 | |
| | **0** | **10** | **20** | **40** | **80** | **[KX01], nM** |

**Table 18**

| **[Calcipotriol], nM** | | | | | | |
|---|---|---|---|---|---|---|
| **1,280** | 66 | 80 | 91 | 97 | 99 | |
| **320** | 72 | 82 | 91 | 97 | 98 | |
| **80** | 71 | 82 | 88 | 95 | 97 | |
| **20** | 59 | 72 | 82 | 93 | 93 | |
| **5** | 56 | 67 | 75 | 88 | 87 | |
| **0** | 53 | 62 | 67 | 84 | 83 | |
| | **0** | **10** | **20** | **40** | **80** | **[KX01], nM** |

Similar studies were performed using a squamous cell carcinoma cell line (SCC A431). Tables 19 and 20 show the results of cell growth inhibition studies (expressed as a percentage relative to control cell growth) for treatment with either KX01 or calcipotriol in combination with exposure to blue light.

**Table 19**

| **Blue light (hours)** | | | | | | |
|---|---|---|---|---|---|---|
| **6** | 66 | 79 | 81 | 81 | 82 | |
| **4** | 45 | 66 | 76 | 73 | 75 | |
| **2** | 40 | 40 | 97 | 97 | 95 | |
| **0** | 0 | 0 | 99 | 99 | 99 | |
| | **0** | **10** | **20** | **40** | **80** | **[KX01], nM** |

Table 19 shows the results of a cell proliferation assay performed using KX01 and blue light and a squamous cell human carcinoma cell line (SCC A431). The percentage of growth inhibition relative to untreated control is shown.

**Table 20**

| **Blue light (hours)** | | | | | | | |
|---|---|---|---|---|---|---|---|
| **6** | 66 | 67 | 69 | 67 | 67 | 68 | |
| **4** | 45 | 49 | 44 | 47 | 49 | 43 | |
| **2** | 18 | 26 | 36 | 41 | 35 | 38 | |
| **0** | 0 | 19 | 30 | 40 | 41 | 36 | |
| | **0** | **5** | **20** | **80** | **320** | **1,280** | **[calcipotrioll, nM** |

Tables 21 to 24 show the effects of combined treatment with KX01, calcipotriol, and exposure to blue light at 0 hours, 2 hours, 4 hours, and 6 hours of light exposure (respectively) on human squamous cell carcinoma cells. Values are expressed as the percentage of growth inhibition relative to cells grown under control conditions (0 hours blue light, [KX01] = 0, [calcipotriol] = 0). The percentage of growth inhibition relative to an untreated control is shown. It should be appreciated that synergy (i.e. greater than additive) effects between blue light exposure and combined KX01 and calcipotriol treatement are observed, for example at 2 to 6 hours of blue light exposure and combined treatment with 10 nM KX01 and 5 nM calcipotriol, and at 2 hours of exposure to blue light and combined treatment with 10 nM KX01 and from 5 to 1,280 nM calcipotriol.

**Table 21**

| **[Calcipotriol], nM** | | | | | | |
|---|---|---|---|---|---|---|
| **1,280** | 36 | 31 | 75 | 96 | 100 | |
| **320** | 41 | 26 | 74 | 98 | 99 | |
| **80** | 40 | 29 | 75 | 97 | 100 | |
| **20** | 30 | 26 | 76 | 96 | 100 | |
| **5** | 19 | 1 | 77 | 96 | 100 | |
| **0** | 0 | 0 | 80 | 99 | 99 | |
| | **0** | **10** | **20** | **40** | **80** | **[KX01], nM** |

**Table 22**

| **[Calcipotriol], nM** | | | | | | |
|---|---|---|---|---|---|---|
| **1,280** | 38 | 55 | 78 | 95 | 98 | |
| **320** | 35 | 56 | 79 | 96 | 98 | |
| **80** | 41 | 56 | 80 | 97 | 98 | |
| **20** | 39 | 52 | 80 | 98 | 98 | |
| **5** | 26 | 46 | 78 | 96 | 98 | |
| **0** | 18 | 40 | 79 | 97 | 95 | |
| | **0** | **10** | **20** | **40** | **80** | **[KX01], nM** |

**Table 23**

| **[Calcipotriol], nM** | | | | | | |
|---|---|---|---|---|---|---|
| **1,280** | 43 | 69 | 75 | 77 | 82 | |
| **320** | 49 | 72 | 77 | 79 | 83 | |
| **80** | 47 | 72 | 78 | 80 | 83 | |
| **20** | 44 | 68 | 74 | 78 | 82 | |
| **5** | 49 | 68 | 74 | 76 | 80 | |
| **0** | 45 | 66 | 69 | 73 | 75 | |
| | **0** | **10** | **20** | **40** | **80** | **[KX01], nM** |

**Table 24**

| **[Calcipotriol], nM** | | | | | | |
|---|---|---|---|---|---|---|
| **1,280** | 68 | 73 | 88 | 93 | 94 | |
| **320** | 67 | 79 | 88 | 91 | 96 | |
| **80** | 67 | 81 | 88 | 89 | 91 | |
| **20** | 69 | 81 | 86 | 88 | 88 | |
| **5** | 67 | 79 | 79 | 84 | 86 | |
| **0** | 66 | 79 | 81 | 81 | 82 | |
| | **0** | **10** | **20** | **40** | **80** | **[KX01], nM** |

As shown, KX01 and calcipotriol in combination exhibit synergistic inhibition of cell growth in keratinocytes *in vitro,* for example within the range of 1 to 30 nM KX01 and 3 to 300 nM calcipotriol. Without wishing to be bound by theory, evidence suggests that at least part of this apparent growth inhibition is due to induction of apoptosis by KX01 and calcipotriol. In addition, blue light shows additive effect on cell growth inhibition in keratinocytes *in vitro* that are exposed to KX01, calcipotriol, and/or a combination of KX01 and calcipotriol, indicating that controlled exposure to blue light can serve as a valuable adjunct to chemotherapies.

Additional studies were performed to evaluate potential synergistic effects of cell growth inhibition of human keratinocytes on exposure to blue light and to KX01 or calcipotriol. Results are shown below in Table 25 and Table 26.

**Table 25**

| **Blue light (hours)** | | | | | | |
|---|---|---|---|---|---|---|
| **6** | 47 | 57 | 73 | 88 | 88 | |
| **4** | 44 | 50 | 68 | 86 | 90 | |
| **2** | 29 | 34 | 68 | 91 | 96 | |
| **0** | 0 | 6 | 49 | 90 | 95 | |
| | **0** | **10** | **20** | **40** | **80** | **[KX01], nM** |

**Table 26**

| **Blue light (hours)** | | | | | | | |
|---|---|---|---|---|---|---|---|
| **6** | 47 | 51 | 58 | 67 | 72 | 67 | |
| **4** | 44 | 47 | 52 | 64 | 67 | 63 | |
| **2** | 29 | 33 | 39 | 53 | 60 | 58 | |
| **0** | 0 | 6 | 17 | 35 | 48 | 37 | |
| | **0** | **5** | **20** | **80** | **320** | **1,280** | **[calcipotriol], nM** |

Synergistic effects between blue light exposure and KX0l or calcipotriol on cell growth inhibition in human keratinocytes were observed. For example, synergy is evident at 6 hours of blue light exposure in combination with 10 nm KX01.

Studies were also performed to evaluate potential synergistic effects of cell growth inhibition of human keratinocytes on exposure to blue light and to KX01 in combination with calcipotriol. Results are shown below in Tables 27 to Table 30, which show results from 0, 2, 4, and 6 hours of blue light exposure (respectively). Values are expressed as the percentage of growth inhibition relative to cells grown under control conditions (0 hours blue light, [KX01] = 0, [calcipotriol] = 0). It should be appreciated that synergy (i.e. greater than additive) effects between blue light exposure and combined KX01 and calcipotriol treatment are observed, and that the synergistic effect observed at 2 hours of blue light exposure and combined treatment with 10 nM KX01 and 20 nM calcipotriol is confirmed.

**Table 27**

| **[Calcipotriol], nM** | | | | | | |
|---|---|---|---|---|---|---|
| **1,280** | 37 | 56 | 85 | 93 | 95 | |
| **320** | 48 | 65 | 91 | 98 | 98 | |
| **80** | 35 | 57 | 91 | 98 | 98 | |
| **20** | 17 | 30 | 86 | 97 | 97 | |
| **5** | 6 | 13 | 72 | 95 | 97 | |
| **0** | 0 | 6 | 49 | 90 | 95 | |
| | **0** | **10** | **20** | **40** | **80** | **[KX01], nM** |

**Table 28**

| **[Calcipotriol], nM** | | | | | | |
|---|---|---|---|---|---|---|
| **1,280** | 58 | 76 | 91 | 96 | 99 | |
| **320** | 60 | 83 | 97 | 100 | 100 | |
| **80** | 53 | 79 | 97 | 100 | 100 | |
| **20** | 39 | 63 | 91 | 99 | 100 | |
| **5** | 33 | 42 | 83 | 97 | 99 | |
| **0** | 29 | 34 | 68 | 91 | 96 | |
| | **0** | **10** | **20** | **40** | **80** | **[KX01], nM** |

**Table 29**

| **[Calcipotriol], nM** | | | | | | |
|---|---|---|---|---|---|---|
| **1,280** | 63 | 76 | 90 | 95 | 96 | |
| **320** | 67 | 80 | 94 | 98 | 98 | |
| **80** | 64 | 76 | 90 | 98 | 98 | |
| **20** | 52 | 61 | 82 | 93 | 97 | |
| **5** | 47 | 54 | 74 | 90 | 94 | |
| **0** | 44 | 50 | 68 | 86 | 90 | |
| | **0** | **10** | **20** | **40** | **80** | **[KX01], nM** |

**Table 30**

| **[Calcipotriol], nM** | | | | | | |
|---|---|---|---|---|---|---|
| **1,280** | 67 | 79 | 90 | 95 | 96 | |
| **320** | 72 | 81 | 93 | 96 | 97 | |
| **80** | 67 | 78 | 90 | 95 | 97 | |
| **20** | 58 | 68 | 84 | 93 | 94 | |
| **5** | 51 | 61 | 80 | 92 | 91 | |
| **0** | 47 | 57 | 73 | 88 | 88 | |
| | **0** | **10** | **20** | **40** | **80** | **[KX01], nM** |

Similar studies were performed to determine the synergistic effects on cell growth inhibition of squamous cell carcinoma cells (SCC A431) on exposure to blue light and KX01 or calcipotriol. Results are shown below in Tables 31 and 32, which show results for KX01 and calipotriol (respectively). Values are expressed as the percentage of growth inhibition relative to cells grown under control conditions (0 hours blue light, [KX01] = 0, [calcipotriol] = 0).

**Table 31**

| **Blue light (hours)** | | | | | | |
|---|---|---|---|---|---|---|
| **6** | 65 | 77 | 83 | 86 | 88 | |
| **4** | 48 | 66 | 74 | 80 | 84 | |
| **2** | 23 | 44 | 83 | 99 | 98 | |
| **0** | 0 | 11 | 79 | 100 | 99 | |
| | **0** | **10** | **20** | **40** | **80** | **[KX01], nM** |

**Table 32**

| **Blue light (hours)** | | | | | | | |
|---|---|---|---|---|---|---|---|
| **6** | 65 | 62 | 63 | 61 | 60 | 58 | |
| **4** | 48 | 50 | 48 | 50 | 51 | 48 | |
| **2** | 23 | 27 | 36 | 40 | 37 | 39 | |
| **0** | 0 | 14 | 19 | 27 | 31 | 28 | |
| | **0** | **5** | **20** | **80** | **320** | **1,280** | **[calcipotriol], nM** |

Synergistic effects between blue light exposure and KX01on cell growth inhibition in squamous cell carcinoma cells are evident, for example at 10 nM KX01.

Studies were also performed to evaluate potential synergistic effects of cell growth inhibition of human squamous cell carcinoma cells on exposure to blue light and to KX01 in combination with calcipotriol. Results are shown below in Tables 33 to Table 36, which show results from 0, 2, 4, and 6 hours of blue light exposure (respectively). Values are expressed as the percentage of growth inhibition relative to cells grown under control conditions (0 hours blue light, [KX01] = 0, [calcipotriol] = 0). It should be appreciated that synergy (i.e. greater than additive) effects between blue light exposure and combined KX01 and calcipotriol treatment are observed, for example at 2 to 6 hours of blue light exposure and combined treatment with 10 nM KX01 and 5 nM calcipotriol, and at 2 hours of exposure to blue light and combined treatment with 10 nM KX01 and from 5 to 1,280 nM calcipotriol are confirmed.

**Table 33**

| **[Calcipotriol], nM** | | | | | | |
|---|---|---|---|---|---|---|
| **1,280** | 28 | 29 | 70 | 97 | 100 | |
| **320** | 31 | 29 | 69 | 97 | 99 | |
| **80** | 26 | 28 | 70 | 97 | 100 | |
| **20** | 19 | 23 | 72 | 97 | 100 | |
| **5** | 14 | 10 | 74 | 97 | 100 | |
| **0** | 0 | 11 | 79 | 100 | 99 | |
| | **0** | **10** | **20** | **40** | **80** | **[KX01], nM** |

**Table 34**

| **[Calcipotriol], nM** | | | | | | |
|---|---|---|---|---|---|---|
| **1,280** | 39 | 57 | 79 | 96 | 98 | |
| **320** | 37 | 56 | 78 | 97 | 98 | |
| **80** | 40 | 55 | 79 | 98 | 99 | |
| **20** | 36 | 51 | 81 | 99 | 99 | |
| **5** | 27 | 45 | 81 | 98 | 99 | |
| **0** | 23 | 44 | 83 | 99 | 98 | |
| | **0** | **10** | **20** | **40** | **80** | **[KX01], nM** |

**Table 35**

| **[Calcipotriol], nM** | | | | | | |
|---|---|---|---|---|---|---|
| **1,280** | 48 | 66 | 74 | 81 | 88 | |
| **320** | 51 | 67 | 76 | 81 | 87 | |
| **80** | 50 | 67 | 76 | 81 | 88 | |
| **20** | 48 | 66 | 74 | 81 | 87 | |
| **5** | 49 | 66 | 76 | 81 | 87 | |
| **0** | 48 | 66 | 74 | 80 | 84 | |
| | **0** | **10** | **20** | **40** | **80** | **[KX01], nM** |

**Table 36**

| **[Calcipotriol], nM** | | | | | | |
|---|---|---|---|---|---|---|
| **1,280** | 57 | 70 | 89 | 96 | 96 | |
| **320** | 60 | 74 | 89 | 94 | 95 | |
| **80** | 61 | 75 | 88 | 92 | 94 | |
| **20** | 63 | 76 | 87 | 91 | 93 | |
| **5** | 62 | 75 | 83 | 89 | 91 | |
| **0** | 65 | 77 | 83 | 86 | 88 | |
| | **0** | **10** | **20** | **40** | **80** | **[KX01], nM** |

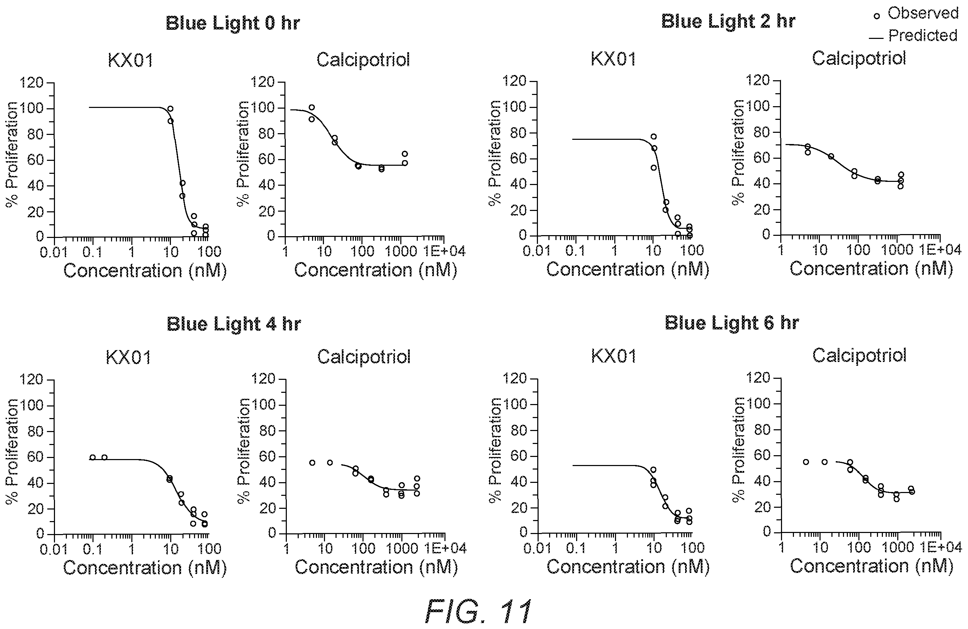

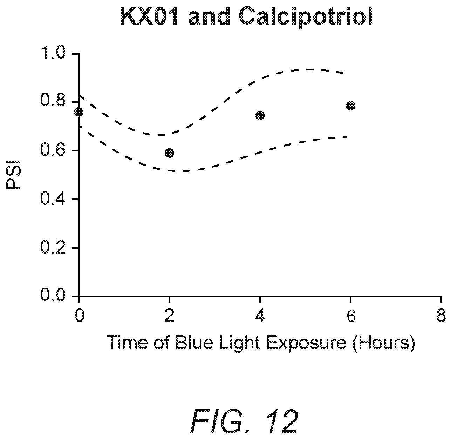

Additional studies were performed to determine PSI values for the inhibition of skin cell proliferation by blue light exposure in combination with KX01 and/or calcipotriol exposure. FIG. 11 shows results of keratinocyte cell proliferation on exposure to either KX01 or calcipotriol at various concentrations and exposure to blue light for from 0 to 6 hours. FIG. 12 shows the results of calculations of PSI values for combined treatment with KX01, calcipotriol, and blue light exposure, where a value of less than 1 indicates a synergistic effect. Synergy between KX01 and calcipotriol is evident in the absence of blue light (0 hours), but additional synergy (as indicated by a further reduced psi value) is seen on exposure to blue light with this drug combination. This is particularly evident at 2 hours of blue light exposure. Table 37 provides a summary of the numeric data presented in FIG. 12.

**Table 37**

| **Time (hours)** | **PSI (95% confidence interval)** |
|---|---|
| **0** | 0.762 (0.690-0.828) |
| **2** | 0.591 (0.514-0.668) |
| **4** | 0.746 (0.595-0.897) |
| **6** | 0.786 (0.660-0.913) |

Inventors believe that additional synergistic effects between exposure to blue light and KX01 and/or calcipotriol can be realized through optimization of the wavelength of blue light utilized, spectral range of the blue light utilized, and/or light intensity.

The compositions disclosed herein can treat dermatological hyperproliferative conditions, including malignant or benign hyperproliferative disorders. Malignant hyperproliferative epidermal pathologies include: squamous-cell carcinoma (SCC), basal-cell carcinoma (BCC) and other non-melanoma skin cancers (NMSCs). Benign hyperproliferative epidermal pathologies include psoriasis, other disturbances of keratinization, common warts, keratoacanthoma, seborrhoeic keratosis, seborrhea and ichthyosis. Contemplated conditions that may be treated using compositions disclosed herein includes: solar keratosis, ichthyosis, Grover's disease, common warts, keratoacanthoma, seborrhoeic keratosis, scleroderma, seborrhea, HIV-associated dermatoses, hyperproliferation associated with wound healing, chronic dermatoses of autoimmune type (e.g., scleroderma and pemphigus vulgaris), alopecia, skin atrophy, photoageing, keratodermos, acne vulgaris, acne rosacea, lichen planus, cutaneous lupus erythematosus, pre-malignant conditions (e.g., melanocytic naevus, myelodysplastic syndrome, among others), Danders disease, palmoplanter keratodermas, pityriasis rubra pilaris, epidermal naevoid syndromes, erythrokeratoderma variabilis, epidermolytic hyperkeratoses, non-bullous ichthyosiform erythroderma, cutaneous lupus erythematosus, and lichen planus.

## Claims

1. A pharmaceutical composition for use in the treatment of a hyperproliferative skin disorder, comprising:
a first compound, wherein the first compound is KX01 and has the formula: 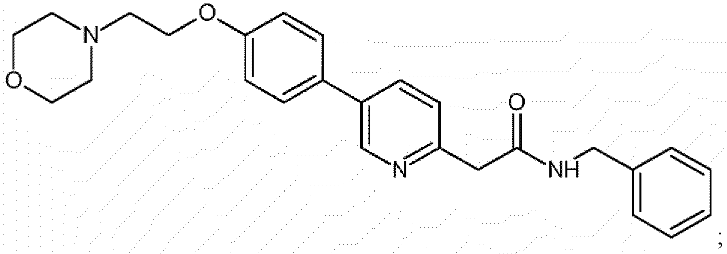 and
a second compound comprising calcipotriol.

2. The pharmaceutical composition for use according to claim 1, wherein the composition is formulated to provide a molar ratio of the first compound to the second compound of 160:3 to 5:243, wherein said ratio provides a synergistic effect at the affected area that exceeds the additive effects of the first compound and the second compound when applied individually at corresponding concentrations.

3. The pharmaceutical composition for use according to any one of claims 1 to 2, wherein the composition further comprises a retinoid.

4. A topical preparation for use in combination with exposure to a blue light in treating a proliferative skin condition, wherein the topical preparation comprises a first compound, wherein the first compound is KX01, and the blue light comprises a wavelength from 280nm to 500 nm, wherein the topical preparation includes the first compound in a first amount that provides a first reduction in proliferation, wherein the blue light is selected to provide a second reduction in proliferation, wherein use of the topical preparation in combination with the blue light provides a combined reduction in proliferation that is greater than the sum of a first reduction in proliferation and the second reduction in proliferation, wherein the proliferative skin condition is actinic keratosis or psoriasis.

5. The topical preparation for use of claim 4, wherein the topical preparation further comprises calcipotriol.

## Patentansprüche

1. Pharmazeutische Zusammensetzung für die Verwendung bei der Behandlung einer hyperproliferativen Hauterkrankung, umfassend:
eine erste Verbindung, wobei die erste Verbindung KX01 ist und die Formel aufweist: 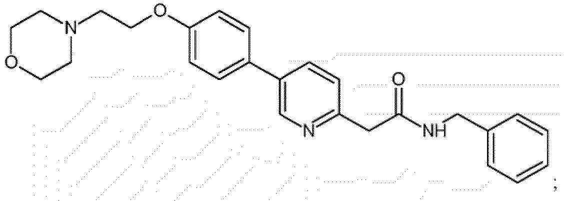 und
eine zweite Verbindung, die Calcipotriol umfasst.

2. Pharmazeutische Zusammensetzung für die Verwendung nach Anspruch 1, wobei die Zusammensetzung formuliert ist, um ein molares Verhältnis der ersten Verbindung zu der zweiten Verbindung von 160 : 3 bis 5 : 243 bereitzustellen, wobei das Verhältnis an der betroffenen Stelle eine synergistische Wirkung bereitstellt, die über die additiven Wirkungen der ersten Verbindung und der zweiten Verbindung hinausgeht, wenn einzeln in entsprechenden Konzentrationen angewendet.

3. Pharmazeutische Zusammensetzung für die Verwendung nach einem der Ansprüche 1 bis 2, wobei die Zusammensetzung ferner ein Retinoid umfasst.

4. Topisches Präparat für die Verwendung in Kombination mit der Aussetzung an blaues Licht bei der Behandlung einer proliferativen Hauterkrankung, wobei das topische Präparat eine erste Verbindung umfasst, wobei die erste Verbindung KX0 ist, und das blaue Licht eine Wellenlänge von 280 nm bis 500 nm umfasst, wobei das topische Präparat die erste Verbindung in einer ersten Menge beinhaltet, die eine erste Reduktion der Proliferation bereitstellt, wobei das blaue Licht ausgewählt ist, um eine zweite Reduktion der Proliferation bereitzustellen, wobei die Verwendung des topischen Präparats in Kombination mit dem blauen Licht eine kombinierte Reduktion der Proliferation bereitstellt, die größer ist als die Summe aus einer ersten Reduktion der Proliferation und der der zweiten Reduktion der Proliferation, wobei die proliferative Hauterkrankung Strahlenkeratose oder Psoriasis ist.

5. Topisches Präparat für die Verwendung nach Anspruch 4, wobei das topische Präparat ferner Calcipotriol umfasst.

## Revendications

1. Composition pharmaceutique destinée à être utilisée dans le traitement d'un trouble cutané hyperprolifératif, comprenant :
un premier composé, dans laquelle le premier composé est KX01 et présente la formule : 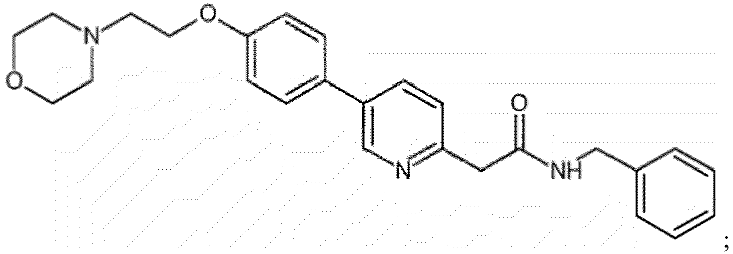 et
un deuxième composé comprenant du calcipotriol.

2. Composition pharmaceutique destinée à être utilisée selon la revendication 1, dans laquelle la composition est formulée pour fournir un rapport molaire entre le premier composé et le deuxième composé de 160:3 à 5:243, dans laquelle ledit rapport produit, au niveau de la région affectée, un effet synergique qui dépasse les effets additifs du premier composé et du deuxième composé lorsqu'ils sont appliqués individuellement à des concentrations correspondantes.

3. Composition pharmaceutique destinée à être utilisée selon l'une quelconque des revendications 1 et 2, dans laquelle la composition comprend en outre un rétinoïde.

4. Préparation topique destinée à être utilisée en combinaison avec une exposition à une lumière bleue dans le traitement d'une affection cutanée proliférative, dans laquelle la préparation topique comprend un premier composé, dans laquelle le premier composé est KX01, et la lumière bleue comprend une longueur d'onde de 280 nm à 500 nm, dans laquelle la préparation topique comporte le premier composé en une première quantité qui assure une première réduction de la prolifération, dans laquelle la lumière bleue est choisie pour assurer une deuxième réduction de la prolifération, dans laquelle l'utilisation de la préparation topique en combinaison avec la lumière bleue assure une réduction combinée de la prolifération qui est supérieure à la somme d'une première réduction de la prolifération et de la deuxième réduction de la prolifération, dans laquelle l'affection cutanée proliférative est la kératose actinique ou le psoriasis.

5. Préparation topique destinée à être utilisée selon la revendication 4, dans laquelle la préparation topique comprend en outre du calcipotriol.
